(19) **Europäisches Patentamt / European Patent Office / Office européen des brevets**

(11) **EP 3 484 372 B1**

(12) **EUROPEAN PATENT SPECIFICATION**

(45) Date of publication and mention
of the grant of the patent:
**09.12.2020 Bulletin 2020/50**

(51) Int Cl.:
*A61B 5/00* *(2006.01)*      *A61B 10/00* *(2006.01)*
*A61B 5/08* *(2006.01)*      *A61B 5/024* *(2006.01)*

(21) Application number: **17739969.8**

(22) Date of filing: **13.07.2017**

(86) International application number:
**PCT/EP2017/067731**

(87) International publication number:
**WO 2018/011357 (18.01.2018 Gazette 2018/03)**

(54) **METHOD AND SYSTEM TO NOTIFY FEMALE FERTILITY PERIOD**

VERFAHREN UND SYSTEM ZUR MELDUNG DES WEIBLICHEN FRUCHTBARKEITSZEITRAUMS

PROCÉDÉ ET SYSTÈME DE NOTIFICATION DE PÉRIODE DE FERTILITÉ CHEZ LA FEMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.07.2016   US 201615209221**

(43) Date of publication of application:
**22.05.2019   Bulletin 2019/21**

(73) Proprietor: **Withings**
**92130 Issy les Moulineaux (FR)**

(72) Inventors:
• **BUARD, Nadine**
**92190 Meudon (FR)**
• **YANG, Rui-Yi**
**78310 Coignieres (FR)**

• **BESNARD, Marc**
**75006 Paris (FR)**
• **HUTCHINGS, Cédric**
**Brookline**
**Massachusetts 02446 (US)**
• **BAILLERGEAU, Matthieu**
**75013 Paris (FR)**

(74) Representative: **Plasseraud IP**
**66, rue de la Chaussée d'Antin**
**75440 Paris Cedex 09 (FR)**

(56) References cited:
**WO-A1-2013/171799      WO-A1-2015/150434
WO-A1-2016/131630      JP-A- 2006 094 969
US-A1- 2016 015 315      US-A1- 2016 058 428**

EP 3 484 372 B1

**Description**

**FIELD OF THE INVENTION**

The present invention relates to methods and systems for determining a fertility period for a female human individual (U).

**BACKGROUND OF THE DISCLOSURE**

There is a need to try to predict and/or notify the fertile periods of a female subject, because in some cases nowadays it may be difficult, for various reasons, to start a pregnancy.
Many methods to predict fertile periods have been proposed, in which the female subject has to take particular measurement(s) at particular moment(s), at wakeup or during the day. However, practically, these methods prove to be annoying and discourage many.
Some have proposed to determine female menstrual cycles by measuring precisely every morning at wakeup a body core temperature of a subject. The menstrual cycle and ovulation dates are estimated by comparing the day temperature value with a monthly average reference value and/or by drafting timing charts, which often upsets the user.
Also are known documents WO2015150434 and US20160058428.
Therefore, there remains a need to propose a system and a method which are particularly non-invasive and unobtrusive, and therefore well accepted by the user. Reliability and simplicity of use is also a target to meet.

**SUMMARY OF THE DISCLOSURE**

The scope of the invention is defined by the independent claims 1 and 12. Preferred features are defined in the dependent claims. According to a first aspect of the present disclosure, it is disclosed a method for determining a fertility period for a female human individual (U), carried out in a system comprising a controller (2), one or more non-invasive sensor(s) (4) configured to sense heart pulse signals of the individual, at least during the sleep of the individual, the method comprising the steps:

/a1/ receiving the heart pulse signals and determine a heart rate (HR),
/b1/ extracting, at the controller, minimal heart rate MSHR(i), over a daily time period of interest T(i), said time period of interest being comprised within a 24-hour period, i denoting a daily index,
/c1/ determine, with values over the past days, a minimal heart rate curve,
/d1/ calculate a characteristic feature in the minimal heart rate curve,
/e/ deducing a current ovulation probability index, according to the characteristic feature in minimal heart rate curve,
/f/ if the ovulation probability index is higher than a predefined threshold denoted PTH, notify the individual with a fertility time window.

Here the term "one or more non-invasive sensor(s)" is to be construed as encompassing any user-worn device, for example a watch-like device, but also devices not worn by the user, like a sensing mat placed in the bed of the user.
Thanks to these dispositions, the fertility time window can be notified to the user very easily, without having the need to monitor a particular biometric parameter from the user standpoint. In practice, the user has just to wear a particular wearable device or sleep in her bed, without bothering about any particular monitoring. This proposed method proves to be unexpectedly simple to use and user-friendly.
In various embodiments of the first aspect of the present disclosure, one may possibly have recourse in addition to one and/or other of the following arrangements.
Prior to step /d1/ there may be provided:

/s1/ normalize the minimal heart rate curve versus a long term heart rate average LTHR specific to the human individual, and step /d1/ is thus changed to:
/d1/ calculate a characteristic feature in the normalized minimal heart rate curve.

Thereby, characteristic feature is made more independent from the user specific parameters; detection is rendered more reliable.
Further, the method may further comprise the steps:

/a2/ determine, from a plurality of heart pulse signals, one or more heart rate variability (HRV) values,
/b2/ extracting, at the controller, one or more HRV index denoted HRVInd(i), over the daily time period of interest T(i),

*/c2/* determine, with values over the past days, one or more HRV index curve(s),

*/d2/* calculate a characteristic feature in the one or more HRV index curve.

With optionally prior to step /d2/ there may be provided:

*/s2/* normalize each of the curves versus a long term average (LTHRV) specific to the human individual, and step /d2/ is thus changed to:

*/d2/* calculate a characteristic feature in the one or more normalized HRV index curve. Further, the one or more non-invasive sensor(s) (4) may be configured to sense respiration signals representative of the respiration cycles of the individual and the method may further comprise the steps:

*/a3/* receiving respiration cycle signals and determine a respiration rate (BR),

*/b3/* extracting, at the controller, average respiration rate ABR(i), during the daily time period of interest T(i), said time period being comprised within a 24-hour period,

*/c3/* determine, over the past days, an average respiration rate curve,

*/d3/* calculate a characteristic feature in the average respiration rate curve

With optionally prior to step /d3/ there may be provided:

*/s3/* normalize the average respiration rate curve versus a long term average LTBR specific to the human individual, and step /d3/ is thus changed to:

*/d3/* calculate a characteristic feature in the normalized average respiration rate curve.

At step */b1/,* the minimal heart rate MSHR(i) may be taken as the lowest value of heart rate during the deep sleep phase(s) of the lapsed night. 'deep sleep' is to be understood as commonly medically known, i.e. the period of sleep produces slow waves with a frequency of less than 1 Hz and a relatively high amplitude.

At step */b1/,* the minimal heart rate MSHR(i) may be taken as the lowest value of heart rate during the complete sleep phase of the lapsed night.

At step */b3/,* the average respiration rate BR(i) may be taken as the average value of respiration rate over the complete sleep phase of the lapsed night.

Generally speaking, at step */e/,* the ovulation probability index is calculated as a weighted combination of characteristic features calculated from each involved curve, namely minimal heart rate curve (possibly after normalization) one or more HRV index curve(s) (possibly after normalization) average respiration rate curve (possibly after normalization).

According to second aspect not forming part of the present invention, it is disclosed a **method** for determining a fertility period for a female human individual (U), carried out in a device comprising a controller (2), one or more non-invasive sensor(s) (4) configured to sense respiration signals representative of the respiration cycles of the individual, at least during the sleep of the individual,

the method comprising the steps:

*/a3/* receiving respiration cycle signals and determine a respiration rate (BR),

*/b3/* extracting, at the controller, average respiration rate ABR(i), during a daily time period of interest T(i), said time period being comprised within a 24-hour period, **i** denoting a daily index,

*/c3/* determine over the past days an average respiration rate curve,

*/d3/* calculate a characteristic feature in the average respiration rate curve,

/e/ deducing therefrom a current ovulation probability index, according to the detection of the characteristic feature in the curve,

*/f/* if the ovulation probability index is higher than a predefined threshold denoted PTH, notify the individual with a fertility time window.

In various embodiments of the second aspect of the present disclosure, one may possibly have recourse in addition to one and/or other of the following arrangements. Prior to step /d3/ there may be provided:

*s3/* normalize the average respiration rate curve versus a long term average LTBR specific to the human individual, and step /d3/ is thus changed to:

*/d3/* calculate a characteristic feature in the normalized average respiration rate curve.

Further, the one or more non-invasive sensor(s) (4) may be configured to sense heart pulse signals of the individual and the method may further comprise the steps:

/a1/ receiving heart pulse signals and determine the heart rate (HR),
/b1/ extracting, at the controller, minimal heart rate MSHR(i), over a daily time period of interest T(i), said time period of interest being comprised within a 24-hour period, **i** denoting a daily index,
/c1/ determine, with values over the past days, a minimal heart rate curve,
/d1/ calculate a characteristic feature in the minimal heart rate curve.

With optionally prior to step /d1/ there may be provided:

/s1/ normalize the minimal heart rate curve versus a long term heart rate average LTHR specific to the human individual, and step /d1/ is thus changed to:
/d1/ calculate a characteristic feature in the normalized minimal heart rate curve.

Further, the method may further comprise the steps:

/a2/ determine, from a plurality of heart pulse signals, one or more heart rate variability (HRV) values,
/b2/ extracting, at the controller, one or more HRV index denoted HRVInd(i), over the daily time period of interest T(i),
/c2/ determine, with values over the past days, one or more HRV index curve(s),
/d2/ calculate a characteristic feature in the one or more HRV index curve

With optionally prior to step /d2/ there may be provided:

/s2/ normalize each of the curves versus a long term average (LTHRV) specific to the human individual, and step /d2/ is thus changed to:
/d2/ calculate a characteristic feature in the one or more normalized HRV index curve

The daily time period of interest may be defined to be a sleep phase of the individual, the sleep phase being determined when a sensed motion sensed by the motion sensor is below a predefined level (MTH).
The one or more non-invasive sensor comprises a wrist worn device placed in contact with or opposite to a portion of the skin of the individual and using a photoplethysmographic technique PPG, configured to sense heart pulses of the individual.
The one or more non-invasive sensor comprises a sensing mat in a bed.
The one or more non-invasive sensor comprises a video camera.
According to a further aspect of the present disclosure, at step generally denoted /d/, the method might combine any of the following three parameters [heart rate, heart rate variability, respiration rate] together to calculate characteristic features therefrom and further determine an ovulation probability index (step /e/), so as to notify the individual with a fertility time window.
According to a further aspect of the present disclosure, it is disclosed a method for determining a fertility period for a female human individual (U), carried out in device comprising a controller, at least a non-invasive heart sensor placed in contact or opposite to a portion of the skin of the individual, at least during the sleep of the individual, the heart sensor being configured to sense heart pulses of the individual and the controller being configured to determine the heart rate ('HR'), the method comprising the following steps:

/a/ collecting, at the sensor, heart pulses signals of the individual,
/b/ extracting therefrom, at the controller, current heart rate during a daily time period of interest, said time period being comprised within a 24-hour period,
/c/ calculate a minimal sleep heart rate denoted MSHR(i), over the daily time period of interest,
/d/ comparing MSHR(i) with values of MSHR(k) recorded previously, and/or comparing MSHR(i) with a long term average of the minimal sleep heart rate values denoted LTHR,
/e/ deducing therefrom a current ovulation probability index, according to a predefined criteria,
/f/ if the ovulation probability index is higher than a predefined threshold, notify the individual with a fertility time window.

Thanks to these dispositions, the fertility time window can be notified to the user very easily, without having the need to monitor a particular biometric parameter from the user standpoint. In practice, the user has just to wear a particular watch-like device or a specific watch at the wrist, without bothering about any particular monitoring. This proposed method proves to be unexpectedly simple to use and user-friendly.
In various embodiments of the invention, one may possibly have recourse in addition to one and/or other of the following arrangements.

According to one option, the heart sensor (4) may be a PhotoPlethysmoGraphic sensor, placed adjacent to a portion of the skin of the individual, and at step /a/ of the method, a PhotoPlethysmoGraphy technique is used. Therefore the proposed method ensures reliability, non invasiveness and unobtrusiveness.

According to one option, the device further comprises a motion sensor (7) and the daily time period of interest is defined to be a sleep phase of the individual, the sleep phase being determined when sensed motion (sensed by the motion sensor) is below a predefined level (MTH).

It was found that performing HR filtering and analysis, particularly on sleep phases, enhances the reliability of the method.

According to one option, at step /e/, the controller identifies a deep sleep phase, and the minimal sleep heart rate ('MSHR') is taken as the lowest value of heart rate during the deep sleep phase(s) of the lapsed night.

It was found that focusing especially on deep sleep phases further enhances the reliability of the method.

According to another option, at step /e/, the controller may take into account a complete sleep phase, and the minimal sleep heart rate ('MSHR') is taken as the lowest value of heart rate during the complete sleep phase(s) of the lapsed night. This way gives also good results.

According to one option, at step /e/, the ovulation probability index is set to exceed PTH if [MSHR(i)- MSHR(i-1) > 0,75 and MSHR(i-1)- MSHR(i-2) > 0,75] or if MSHR(i)- MSHR(i-3) > 2.

It was found that such a substantial increase generally reflects the ovulation process.

According to one aspect, at step /e/ the ovulation probability index is set to exceed PTH if MSHR(i)- LTHR > 0,1 and LTHR - MSHR(i-1) > 0,1.

It was found that such event also generally reflects the ovulation process.

The ambient temperature and/or the skin temperature may also be taken into account in the calculation and/or in the predefined criteria.

One or two particular derating logic calculations can be applied to discard the effects of the ambient temperature and/or the skin temperature.

The present invention also targets a <u>device</u>, intended to be used to notify a female human individual (U) with a fertility time window, comprising a controller (2) and at least a non-invasive heart sensor (4) intended to be placed in contact or opposite to a portion of the skin of the individual, at least during the sleep of the individual, the sensor being configured to sense heart pulses of the individual and the controller being configured to determine the heart rate, the controller being configured to calculate a minimal sleep heart rate denoted MSHR(i), over a daily time period of interest T(i), said time period being comprised within a 24-hour period,

the controller being configured to compare MSHR(i) with values of MSHR recorded the preceding nights, and/or comparing MSHR(i) with a long term average of the minimal sleep heart rate values denoted LTHR,

the controller being configured to determine a current ovulation probability index, according to a predefined criteria, and if the ovulation probability index is higher than a predefined threshold, to notify the individual with a fertility time window.

According to an option, the device may further comprise a motion sensor and the daily time period of interest is defined to be a sleep phase of the individual, the sleep phase being determined when sensed motion is below a predefined level.

According to an option, the heart sensor may be a PhotoPlethysmoGraphic sensor, placed adjacent to a portion of the skin of the individual.

In a particular embodiment, the device is a wristwatch (1) including the non-invasive sensor.

In particular embodiment, the device comprises a display element (3) to display the fertility period notification.

The present invention also targets an **apparatus** for notifying a female human individual (U) with a fertility time window, configured to carry out the method as described is any of the aspects and embodiments described above.

BRIEF DESCRIPTION OF THE DRAWINGS

Other features and benefits of the invention appear from the following detailed description of one of its embodiments, given by way of non-limiting example, and with reference to the accompanying drawings, in which:

- Figure 1 shows a female user sleeping and wearing a monitoring device at the wrist,
- Figure 2 shows a cross section of the monitoring device at the wrist,
- Figure 3 shows a focused cross section of a wrist watch like embodiment,
- Figure 4 is an example time chart showing a typical night sequence with determination of a Minimal Sleep Heart Rate,
- Figure 5 shows a time chart exhibiting the Minimal Sleep Heart Rate over several ovulations cycles,

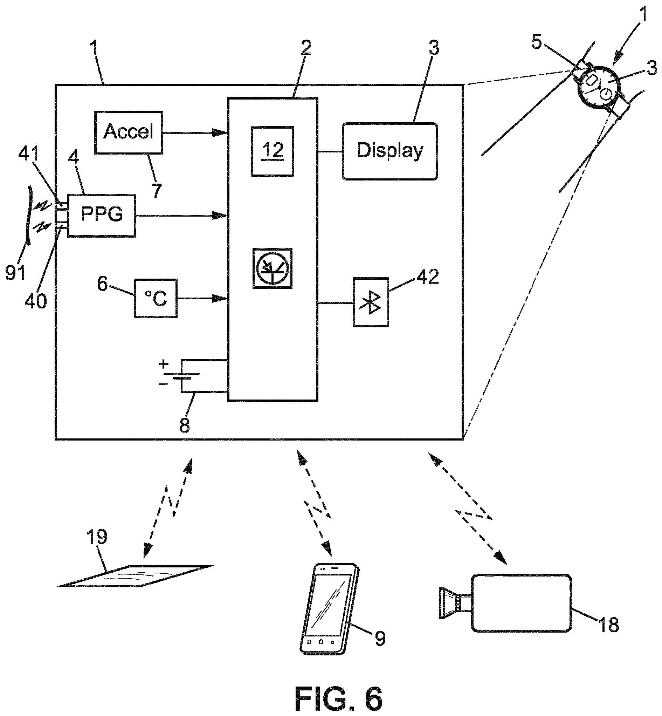

- Figure 6 shows a block diagram of the monitoring device,
- Figure 7 illustrates various steps of variants of the method,
- Figure 8 shows a female user sleeping in a bed equipped with a sensing mat and wearing a monitoring device at the wrist,
- Figure 9 is an example time chart exhibiting the Minimal Sleep Respiration Rate,

- Figure 10 and 11 show examples of time charts exhibiting the Heart Rate Variability indexes.

DETAILED DESCRIPTION OF THE DISCLOSURE

In the figures, the same references denote identical or similar elements.

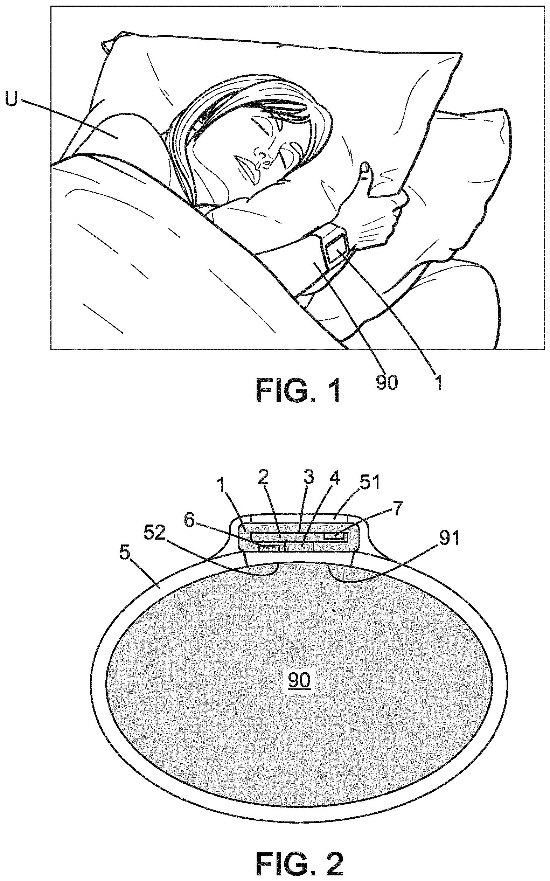

As illustrated on Figures 1 and 2, a first exemplary embodiment of the present disclosure, shown a watch-like device 1.

The watch-like device **1** may be worn at the wrist **90,** via a wrist strap **5.** Said wrist strap **5** may have a front window **51** through which user can view the display of the watch-like device and a back window **52** that will be discussed later.

The watch-like device can be a conventional wrist watch, an activity monitor otherwise called activity tracker, a smart watch, or the like.

The watch-like device **1** comprises a control unit **2** otherwise called processing unit 2 or CPU.

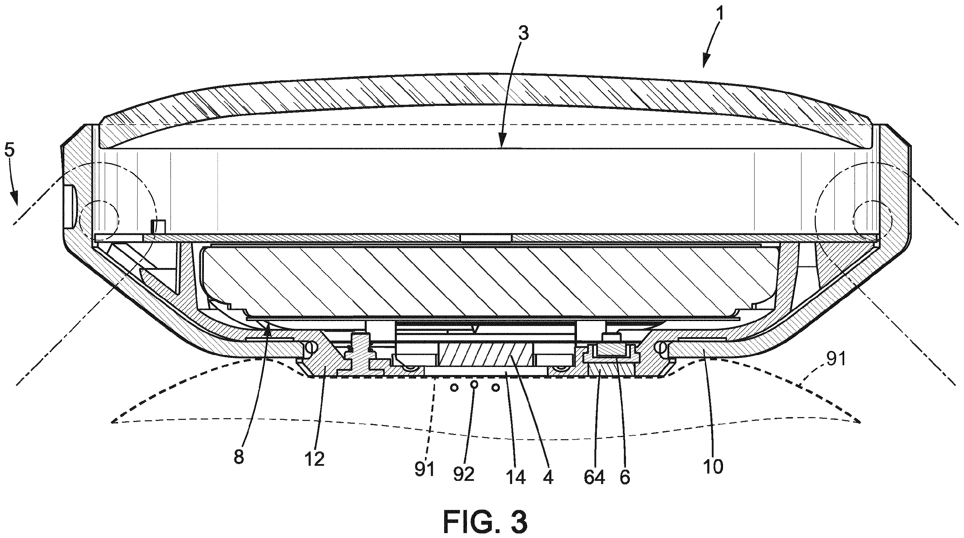

According to the exemplified embodiment illustrated at Fig 3, a non-invasive heart sensor **4** is placed in contact with a portion of the skin **91** of the individual, behind a protective glass **14.**

Alternatively, as shown at Fig 2, the non-invasive heart sensor **4** can be placed opposite to the skin, at a certain distance from the skin.

The non-invasive heart sensor **4** is configured to sense heart pulse signals of the individual **U.**

In an embodiment, the non-invasive heart sensor **4** is a PhotoPlethysmoGraphic sensor, placed adjacent to the skin. As known per se, in one solution among others, an infrared source **41** illuminates a skin portion and a photoreceptor **40** senses the light emitted, which fluctuates in accordance with the flow of blood.

Whether the non-invasive heart sensor is in direct contact or not with the skin, it can sense the blood circulation in small capillary artery/vein network denoted **92** (Fig 3).

Generally, the one or more non-invasive sensor(s) can comprise any user-worn device, besides the example a watch-like device, a finger smart ring, an ear plug, a head band, slippers, and so on. Also the one or more non-invasive sensor(s) can comprise devices not worn by the user, like a sensing mat placed in the bed of the user, a video camera.

However, in other embodiments, the non-invasive heart sensor **4** can be a miniaturized IR video camera using IR PhotoPlethysmoGraphic technique, contfigured to sense the heart pulses at a certain distance from the skin. A IR video camera using a IR PhotoPlethysmoGraphic technique is taught in document EP2976998 ("*Baby Monitor IR*").

However, in other embodiments, the non-invasive heart sensor **4** can be an impedancemetry sensor placed adjacent to the skin of the subject.

According to another variant, the non-invasive heart sensor **4** can be a ballistography sensor, like for example a sensing mat disposed within the bed, as disclosed in document EP2873368 ("*sensing mat*").

Additionally, a motion sensor **7** may be provided in the activity monitor on order to sense the accelerations and movements of the user. The motion sensor is formed in the illustrated example as a multi-axis acceleration sensor **7** (also called 'accelerometer'). The processing unit **2** samples the signals outputted by the acceleration sensors. The processing unit **2** computes said signals in order to assess user acceleration, movements and other information as this will be discussed below

The activity monitor may also comprise a temperature sensor **6.** In some body locations, the temperature sensor 6 is intended to be close to the body of the user in order to sense an environmental temperature close to the user's skin.

For example, as illustrated on figure 2, the temperature sensor **6** can be located at the back side of the device, opposite to the display **3,** and thanks to the back window of the wrist strap **5,** the temperature sensor is facing the skin of the user at the user's wrist **90.** As illustrated on figure 3, the temperature sensor **6** can be arranged behind a thermally conductive cover **64.**

Further, there may be provided an ambient air temperature (not shown), configured to sense the general environment temperature and which may differ from the skin temperature.

Heart pulse signals can also be used to compute one or more HRV index denoted HRVInd(i), over the daily time period of interest T(i), **i** denoting a daily index.

The non-invasive heart sensor **4** may also be able to sense respiration signals representative of the respiration cycles of the individual. The controller can therefrom determine a respiration rate (BR), can extract an average respiration rate denoted **BR(i).**

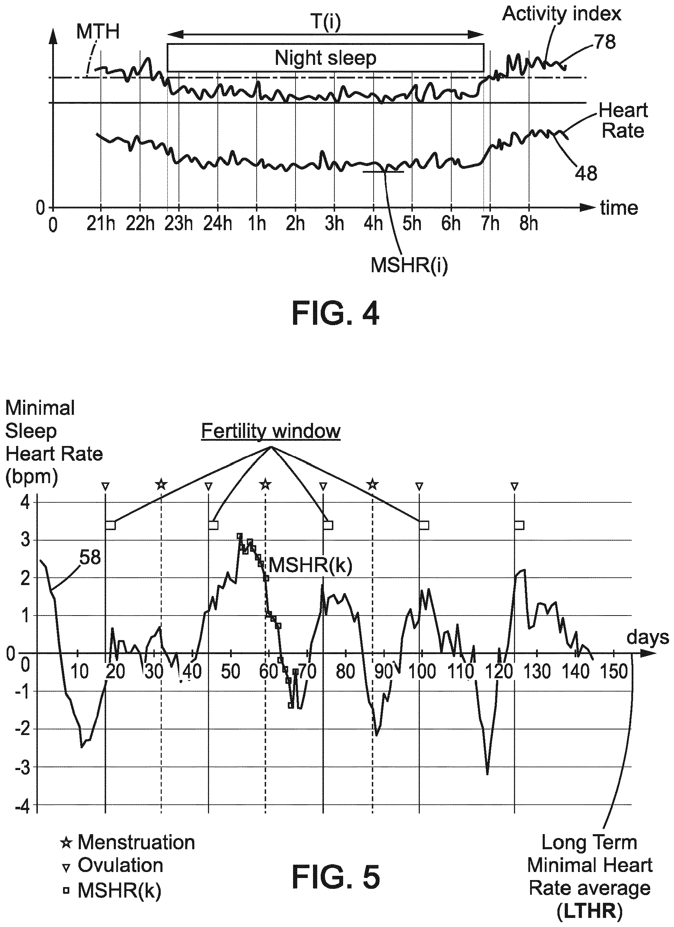

As illustrated on figures 4 and 5, the monitoring device 1 monitors the Heart Rate of the female subject over time. During the sleep, the heart sensor is used to sense heart pulses of the individual (step /a/ of the method), whatever the technique used (PPG, IPG, ballistography). The controller **2** is configured to determine the heart rate **(HR),** after measuring the time interval between two subsequent heart pulses, (step **/b/** of the method). Each HR sample is recorded and this represents a curve versus time (the lower curve **48** on figure 4).

The heart rate (HR) is quantified in **"bpm"** units, i.e. **b**eats **p**er **m**inute; at rest, generally an adult has a HR between 60 bpm and 80 bpm; a younger subject may have a higher range, e.g. between 70 bpm and 100 bpm.

The curve of the HR exhibits a minimum over the night. The minimal sleep heart rate over the night is denoted **MSHR(i).** In the shown example, this minimum occurs around 4h15.

Generally speaking, the minimum over the past 24 hours can also be considered. The minimal sleep heart rate over past 24 hours period is denoted **MSHR(i),** with no regard of what kind of sleep phases happened. **i** denotes a daily index, i.e. one overall value per day.

Focusing back to the sleep phase(s), sleep state can be determined and/or confirmed through an *'activity index'* obtained from the sensed motion sensed at the motion sensor **7.** As illustrated at Fig 4, the activity index is monitored overt time (top curve **78** on Fig 4), and such activity index is compared with a predefined level denoted **MTH** (for Motion Threshold). When the activity index is below **MTH,** then a state of sleep of user U is assumed. When the activity index turns to exceed **MTH,** then the state of sleep is ended. Sleep state is also assessed through the skin temperature sensor data.

There can be a low-pass filtering on the sensed motion data sensed at the motion sensor **7.**

Then, on a larger time scale, each night **MSHR(i)** is plotted on a curve **58** shown at Fig 5.

The controller is therefore able to compare the most recent MSHR(i) with values of **MSHR(k)** recorded previously. More precisely **MSHR(k)** recorded over the two or three previous days are of particular interest.

The controller is also able to compare the most recent **MSHR(i)** with a long term average of the minimal sleep heart rate values denoted **LTHR.** The long term average **LTHR** can be computed from a general HR average over a rolling window ranging on last month or last 2 months, or even more. **LTHR** reflects the heart rate at complete rest of an individual. This value differs from one individual to another, and therefore it's important that the calculations are referenced to this personal biometric characteristic.

Each MSHR(i) per night is recorded and this represents a curve versus time, one example denoted **58** is given at figure 5.

According to the present invention, characteristics and particulars of this curve are used to detect the ovulation date of the human subject.

More precisely, the control unit is configured to perform calculation about sample points of this curve to output and ovulation probability index.

This calculation can be performed on the raw curve with raw values **MSHR(i)** (i.e. before normalization) or alternately on the 'normalized' curve. Normalization can consist here in subtracting the above-mentioned long term average **LTHR** to the raw values **MSHR(i)** to give what is called the 'normalized' curve with normalized' values **MSHR_n(i).** Normalisation step is denoted **/s1/.** Stated otherwise

$$MSHR\_n(i) = MSHR(i) - LTHR.$$

The ovulation event is declared when ovulation probability index exceeds a predefined threshold denote **PTH.**

More generally speaking, the controller is configured to

**/e/** deduce from the curve of **MSHR(i)** a current ovulation probability index, according to a predefined criteria,
**/f/** if the ovulation probability index is higher than the threshold **PTH,** notify the individual with a fertility time window.

According to one option, the ovulation probability index is set to exceed PTH if [MSHR(i)- MSHR(i-1) > 0,75 and MSHR(i-1)- MSHR(i-2) > 0,75]. This denotes two successive increments.

According to another option, the ovulation probability index is set to exceed PTH if MSHR(i)- MSHR(i-3) > 2. This denotes a general ramp-up criteria. MSHR(i)- MSHR(i-2) > 1,5 is an alternate criterion.

According to another option, the ovulation probability index is set to exceed PTH if MSHR(i)- LTHR > 0,1 and LTHR - MSHR(i-1) > 0,1. This denotes a "zero-crossing" criteria, in other words the curve goes from negative side of LTHR to positive side of LTHR.

The same examples can be computed on raw values **MSHR(i)** or alternately on the 'normalized' values **MSHR_n(i).**

The above mentioned examples are instances of what should be understood generically here by 'calculate a characteristic feature' in a curve. This calculation uses at least the last known value DO of the curve (from last past night) and possibly 1 to 4 values looking backwards in the curve (days D-1, D-2, D-3, D-4). Regarding heart rate HR, this concerns steps /d/ and/or /d1/, and the characteristic feature can be named *'first'* characteristic feature.

We note that the characteristic feature can also be named a *'metric'.*

The watch or activity monitor **1** comprises a display **3** controlled by the processing unit **2.** On the display 3, various information can be made available to the user such a particular color whenever the fertility time window number is "ON".

The monitor device comprises a wireless communication interface **42** (here for example Bluetooth™, or Bluetooth™ Low Energy 'BLE' or the like), for sending collected data to a second device **9** like a smartphone for example. Time charts, histograms and so on can be displayed in a nice fashion on the smartphone screen.

The monitor device **1** is powered by an on-board source of energy **8,** for example a rechargeable battery. This battery supplies all the on-board elements in the device (the sensor **7,** the display 3 and the processing unit 14, etc..). The battery can be a lithium button cell type battery, e.g. a CR2025 battery, providing an autonomy of several months in normal use.

The monitor device **1** measures continuously the user's heart rate, and optionally the HRV and the respiration Rate BR, to determine sport/activity Heart Rate and recovery Heart Rate, daily Heart Rate, resting Heart Rate, etc...

The monitor device **1** provides a 24/7 automatic activity tracker; it automatically detects and analyzes the user everyday moves, whether the user is walking, running, swimming or sleeping.

In one embodiment, the monitor device **1** is water-resistant to 50 meters (5ATM). Regarding its mechanical construction, the wristwatch 1 illustrated at Fig 3 comprises a stainless steel casing **10** with a diameter comprised between 32 mm and 38 mm and a thickness comprised between 10 and 14 mm, for example between 12 mm and 13 mm. The monitor device **1** comprises a cup-like plastic base **12** for housing the battery, the PPG sensor **4** and the temperature sensor **6.**

At the system level, there can be provided beyond the already mentioned smartphone **9,** a sensing mat **19** to be placed in the bed, and a conventional video camera **18.** The smartphone **9** is configured to display data and histograms about the sleep and the female cycle assessment, together with one or more user notification about the abovementioned fertility time window.

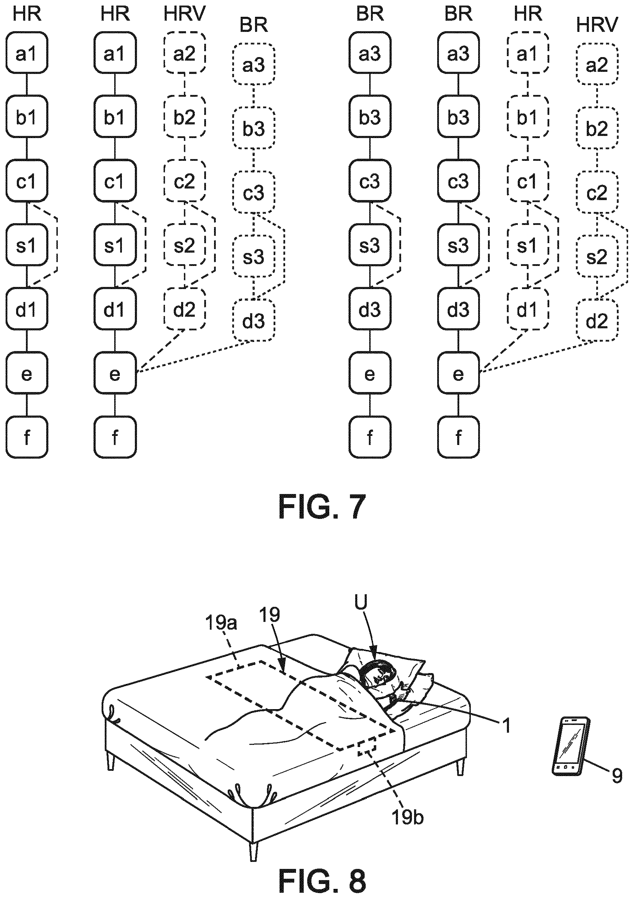

As illustrated on Figure 8, a second exemplary embodiment of the present disclosure, there is shown a bed sensing mat **19.** In the illustrated example, the bed sensing mat **19** may comprise a pneumatic bladder **19a** and a unit **19b** that may be a simple signal conditioner or a more elaborate control unit. The controller can be included in the unit denoted 19b or farther from the bladder, on a bedside device like for example a snooze-like unit intended to be placed close to the bed.

As described in details in EP2873368, the pressure variations sensed by the pneumatic bladder are transduced in electrical signals which reflect the respiration cycle of the user **U** resting on the bed. Said electrical signals also reflect the heart pulses. The sensing mat is thus a non-invasive sensor adapted to sense respiration signals representative of the respiration cycles of the individual, at least during the sleep of the individual.

The controller 2 is configured to perform the following steps:

/a3/ receiving respiration cycle signals and determine a respiration rate (BR),
/b3/ extracting, at the controller, an average respiration rate ABR(i), during a daily time period of interest T(i), said time period being comprised within a 24-hour period, i denoting a daily index,
/c3/ determine over the past days an average respiration rate curve,
/d3/ calculate a characteristic feature in the average respiration rate curve.

According to one example, the average respiration rate **ABR(i)** is taken considering the total sleep phase. According to another example, the average respiration rate **ABR(i)** is taken considering only some particular sleep phase, deep sleep, REM or other.

The characteristic feature taken from the average respiration rate curve can be named here 'third' characteristic feature.

Between steps/**c3/** and /**d3/** there may be added a normalization step.

In the normalization step /**s3/** the controller normalizes the average respiration rate curve versus a long term average LTBR specific to the human individual.

Normalization can consist here in subtracting the above-mentioned long term average of average respiration rate denoted LTBR to the raw values ABR(i) to give what is called the 'normalized' curve with normalized' values **ABR(i)_s**. In such case, step /d3/ is thus changed to /**d3/** calculate a characteristic feature in the normalized average respiration rate curve.

After that, the controller 2 is configured to perform similar steps as per HR, namely

/e/ deducing therefrom a current ovulation probability index, according to the detection of the characteristic feature in the raw or normalized curve,
/f/ if the ovulation probability index is higher than a predefined threshold denoted PTH, notify the individual with a fertility time window.

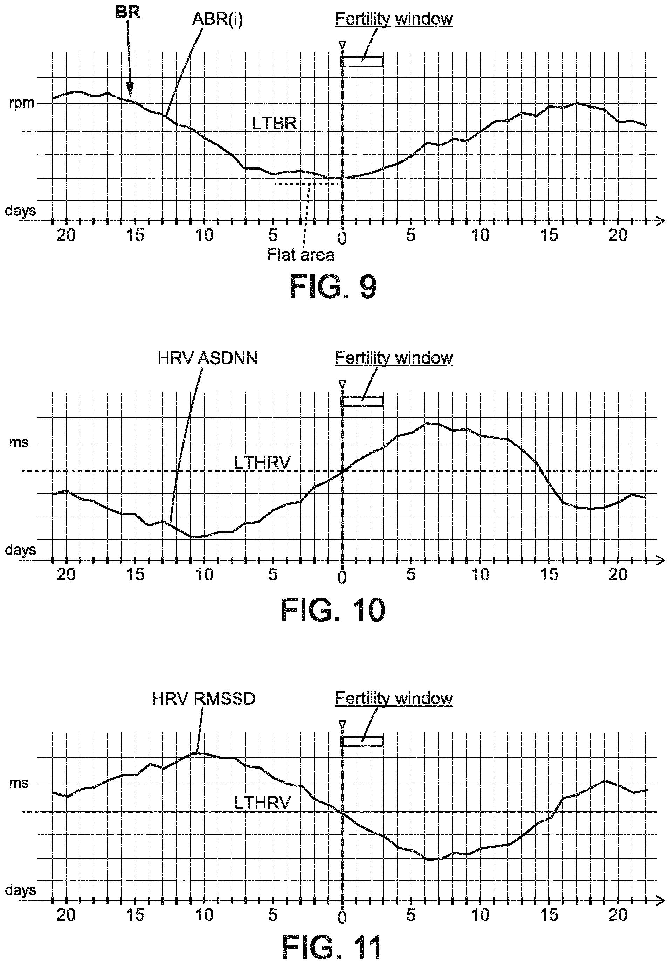

Regarding the characteristic feature in respiration rate curve, one seeks to detect a flat area shown in figure 9, this flat area is a marker of ovulation. The characteristic feature can be defined as a derivative of this curve compared to zero.

Regarding the HRV, the following statement may apply either to the first embodiment or to the second embodiment.

Heart rate variability (HRV) measures the specific changes in time (or variability) between successive heart beats. In particular example, beat time intervals **IBI(i)** is measured in milliseconds (ms) and is taken between successive heart pulses defined by successive heart pulse apexes. $AVHP = \frac{1}{N} \sum_{t1}^{t2} IBI(i)$ denotes the average time interval between heart pulses during the time period between t1 and t2 (N beats).

There is defined standard deviation or a predefined time, for example 5 min. ASDNN is a first HRV Index. ASDNN is defined as follows :

$$ASDNN = \sqrt{\frac{1}{N} \sum_{t1}^{t2} (IBI(i) - AVHP)^2}$$

Where t2-t1 is for example 300 s. AVHP is also computed between t1 and t2.

According to another possibility, such a heart rate variability index HRVI can be expressed by Max **[IBI(i)]** - Min **[IBI(i)]**, where indicia **i** is ranging from 1 to **i0,** i0 being the number of sampled heart beats.

According to another possibility, the heart rate variability index HRV index can be inferred from time parameters such as the Root Mean Squared of the Successive Differences (RMSSD) of **IBI**(i) over several successive heart beats, as follows:

$$RMSSD = \sqrt{\sum_{k1}^{k2-2} \frac{([IBI(i+2) - IBI(i+1)]^2}{k2 - k1 - 1}}$$

The indexes k1 and k2 delimits the time span taken in consideration; it can be 5 minutes, 10 minutes, 30 minutes, 60 minutes.

According to the present disclosure, ASDNN and RMSSD are called generically HRV Indexes, one value HRVInd(i) is issued after each night (each period of interest) and the series of subsequent values are used to define one or more generically HRV Index curves, where HRVInd(i) denotes generically the values output at day **i.**

In order to have the characteristic feature independent from specific individual **U,** these values are normalized with regard to a long term average of heart rate viability **LTHRV.**

The following calculation is made at least over a one complete sleep sequence, or, alternatively, over more than one sleep sequence.

$$LTHRV = \frac{1}{N} \sum_{t0}^{t4} |IBI(i+1) - IBI(i)|$$

Figure 10 show a ASDNN curve together with a long term average.
The characteristic feature is defined as the moment when the ASDNN curve crosses the long term average upwards.
Figure 11 show a RMSSD curve together with a long term average.
The characteristic feature is defined as the moment when the RMSSD curve crosses the long term average downwards.

According to still another possibility, the heart rate variability index HRVI can be expressed by the metric named HR PNN50 : $HRV\ PNN50 = \frac{N1(HRV>50)}{N}$ where **N** is the samples of HRV and **N1** (HRV>50) is the number of samples of HRV **[IBI(i+1)** - IBI(i)] is greater than 50 ms.

With the above example in mind, generically, the controller **(2 or 19b)** is configured to :

/a2/ determine, from a plurality of heart pulse signals, one or more heart rate variability **(HRV)** values,
**/b2/** extracting, at the controller, one or more **HRV index** denoted **HRVInd(i),** over the daily time period of interest T(i),
**/c2/** determine, with values over the past days, one or more **HRV index** curve(s),
**/d2/** calculate a characteristic feature in the one or more HRV index curve.

At step /d2/, the characteristic feature can be named here 'second' characteristic feature.
As per HR and BR, the calculation of the characteristic feature uses at least the last known value **HRVInd(0)** of the curve (from last past night) and possibly 1 to 4 values looking backwards in the curve (days D-1, D-2, D-3, D-4).

There may be provided a normalization step versus LTHRV, the result of which is shown at figures 10 and 11, last days curves 'oscillates' with regard to LTHRV.

Figure 7 summarises values possibilities to use the following three parameters [heart rate, heart rate variability, respiration rate] together to calculate characteristic features therefrom and further determine an ovulation probability index (step /e/), so as to notify the individual with a fertility time window.

When used in combination the ovulation probability index is calculated as a weighted combination of characteristic features calculated from each involved curve.

As an example, the controller **(2 or 19b)** can calculate the derivative from the HRV index curves and the BR index curves as features in addition to the heart rate. Then, to calculate the respective weights in order to calculate the final probability, a linear regression included all selected features is used. The final probability is calculated by summing all the features value weighted by the coefficients found in the previous linear regression. Then, this probability is compared to the probability threshold PTH of the ovulation to determine the beginning of the fertility window.

It should be noted that normalization step (/s1/, /s2/, /s3/) is either present or not present.

It should be noted that HRV calculations here do not require to perform FFT (FFT requires indeed high sampling rate, large memory space and powerful processor), and that HR, BR, HRV calculations do not require to handle a large history database.

One can notice that only the long term values LTHR, LTBR, LTHRV and values from the most recent 5 days are to be kept in memory. The long term averages use digital low pass filtering which does not require to store old values. Only the necessary values are stored in some rolling windows, and old values can be continuously discarded (possibly after remote transmission to smartphone 9).

Thus the proposed method can be carried out in a cost effective user-worn device like a wrist watch, with low electrical consumption and long battery autonomy.

It is noted that it is not excluded to compute one particular characteristic feature or metric only on the past day DO and the long term average, in which case the use of older values from days before D-1, D-2, D-3 are not necessary. Also, in some other cases, characteristic feature or metric are computed with values of the past day DO older values from days before D-1, D-2, D-3, with the long term average being unnecessary.

Fertility window is notified to the user as soon it is positively detected (day 0 at Figures 9-11). Fertility window can be set as a 3 day window. Fertility window can be set as a 4 day window or as a 2 day period. One possibility at the controller is to perform the calculation of the characteristic features and the ovulation probability index once a day in the morning.

It should be noted that the ovulation probability index may also depend on the number of days lapsed since the date of the last previously notified fertility window.

## Claims

1.  **A** method for determining a fertility period for a female human individual (U), carried out in a system comprising a controller (2), one or more non-invasive sensor(s) (4) configured to sense heart pulse signals of the individual, at least during the sleep of the individual,
    the method comprising the steps:

    /a1/ receiving the heart pulse signals and determine a heart rate (HR),
    /b1/ extracting, at the controller, minimal heart rate MSHR(i), over a daily time period of interest T(i), said time period of interest being comprised within a 24-hour period, i denoting a daily index,
    /c1/ determine, with values over the past days, a minimal heart rate curve (58),
    /d1/ calculate a characteristic feature in the minimal heart rate curve,
    /e/ deducing a current ovulation probability index, according at least to the characteristic feature in minimal heart rate curve,
    /f/ if the ovulation probability index is higher than a predefined threshold denoted PTH, notify the individual with a fertility time window.

2.  The method according to claim 1, wherein prior to step /d1/ there is provided:
    /s1/ normalize the minimal heart rate curve versus a long term heart rate average LTHR specific to the human individual,
    and step /d1/ is further defined by:
    the minimal heart rate curve is the normalized minimal heart rate curve.

3.  The method according to claim 1 or claim 2,

the method further comprising the steps:

/a2/ determine, from a plurality of heart pulse signals, one or more heart rate variability (HRV) values,
**/b2/** extracting, at the controller, one or more HRV index denoted HRVInd(i), over the daily time period of interest T(i),
**/c2/** determine, with values over the past days, one or more HRV index curve(s),
**/d2/** calculate a characteristic feature in the one or more HRV index curve.

4. The method according to claim 3, wherein prior to step /d2/ there is provided:

**/s2/** normalize each of the curves versus a long term average (LTHRV) specific to the human individual,
and step /d2/ is further defined by:
the one or more HRV index curve is the one or more normalized HRV index curve.

5. The method according to anyone of the claims 1 to 4, wherein the one or more non-invasive sensor(s) (4) is configured to sense respiration signals representative of the respiration cycles of the individual, the method further comprising the steps:

**/a3/** receiving respiration cycle signals and determine a respiration rate (BR),
**/b3/** extracting, at the controller, an average respiration rate ABR(i), during the daily time period of interest T(i), said time period being comprised within a 24-hour period,
**/c3/** determine, over the past days, an average respiration rate curve,
**/d3/** calculate a characteristic feature in the average respiration rate curve.

6. The method according to claim 5, wherein prior to step /d3/ there is provided
**/s3/** normalize the average respiration rate curve versus a long term average LTBR specific to the human individual, and step /d3/ is further defined by:
the average respiration rate curve is the normalized average respiration rate curve.

7. The method according to anyone of the claims 1 to 6, wherein at step **/b1/,** the minimal heart rate MSHR(i) is taken as the lowest value of heart rate during the deep sleep phase(s) of the lapsed night.

8. The method according to anyone of the claims 5 to 6, wherein at step **/b3/,** the average respiration rate BR(i) is taken as the lowest value of respiration rate during the deep sleep phase(s) of the lapsed night.

9. The method according to anyone of the claims 5 to 6, wherein at step /e/, the ovulation probability index is calculated as a weighted combination of characteristic features calculated from each involved curve.

10. The method according to any of the claims 1 to 9 wherein the daily time period of interest is defined to be a sleep phase of the individual, the sleep phase being determined when a sensed motion sensed by a motion sensor is below a predefined level (MTH).

11. The method according to any of the claims 1 to 10, wherein the one or more non-invasive sensor comprises a wrist worn device placed in contact with or opposite to a portion of the skin of the individual and using a photoplethysmographic technique, configured to sense heart pulses of the individual.

12. An **apparatus** for notifying a female human individual (U) with a fertility time window, configured to carry out the method of any of the claims 1 to 11.

13. The apparatus according to claim 12, wherein the one or more non-invasive sensor comprises a sensing mat in a bed (19).

14. The apparatus according to any of the claims 12 to 13, wherein the one or more non-invasive sensor comprises a video camera (18).

**Patentansprüche**

1. Verfahren zum Bestimmen eines Fruchtbarkeitszeitraums für ein weibliches menschliches Individuum (U), das in einem System durchgeführt wird, das eine Steuereinrichtung (2), einen oder mehrere nichtinvasive Sensor(en) (4) aufweist, um Herzpulssignale des Individuums zumindest während des Schlafs des Individuums zu messen, wobei das Verfahren die Schritte aufweist:

   /a1/ Empfangen der Herzpulssignale und Bestimmen einer Herzfrequenz (HR),
   /b1/ an der Steuerung, Extrahieren einer minimalen Herzfrequenz MSHR(i) über einen interessierenden täglichen Zeitraum T(i), wobei der interessierende Zeitraum in einem 24-Stunden-Zeitraum enthalten ist, i einen Tagesindex bezeichnet,
   /c1/ Bestimmen einer Kurve minimaler Herzfrequenz (58) mit Werten über die vergangenen Tage,
   /d1/ Berechnen eines charakteristischen Merkmals in der Kurve minimaler Herzfrequenz,
   /e/ Deduzieren eines aktuellen Ovulationswahrscheinlichkeitsindexes gemäß zumindest dem charakteristischen Merkmal in der Kurve minimaler Herzfrequenz,
   /f/ wenn der Ovulationswahrscheinlichkeitsindex höher als ein vorab definierter Schwellenwert, PTH genannt, ist, Melden eines Fruchtbarkeitszeitfensters an das Individuum.

2. Verfahren nach Anspruch 1, wobei vor Schritt /d1/ vorgesehen ist:

   /s1/ Normieren der Kurve minimaler Herzfrequenz gegen einen Langzeit-Herzfrequenzdurchschnitt LTHR, der für das menschliche Individuum spezifisch ist,
   und Schritt /d1/ ferner dadurch definiert ist, dass:
   die Kurve minimaler Herzfrequenz die normierte Kurve minimaler Herzfrequenz ist.

3. Verfahren nach Anspruch 1 oder Anspruch 2,
   wobei das Verfahren ferner die Schritte aufweist:

   /a2/ Bestimmen eines oder mehrerer Herzfrequenzvariabilität (HRV) Werte aus einer Vielzahl von Herzpulssignalen,
   /b2/ an der Steuereinrichtung, Extrahieren eines oder mehrerer HRV-Indizes, HRVInd(i) genannt, über den interessierenden täglichen Zeitraum T(i),
   /c2/ Bestimmen einer oder mehrerer HRV-Indexkurve(n) mit Werten über die vergangenen Tage,
   /d2/ Berechnen eines charakteristischen Merkmals in der einen oder den mehreren Indexkurven.

4. Verfahren nach Anspruch 3, wobei vor Schritt /d2/ vorgesehen ist:

   /s2/ Normieren jeder der Kurven gegen einen Langzeitdurchschnitt (LTHVR), der für das menschliche Individuum spezifisch ist,
   und Schritt /d2/ ferner dadurch definiert ist, dass:
   die eine oder mehr HRV-Indexkurve die eine oder mehr normierte HRV-Indexkurve ist.

5. Verfahren nach einem der Ansprüche 1 bis 4, wobei der eine oder die mehreren nichtinvasiven Sensor(en) (4) konfiguriert ist/sind, Atmungssignale zu detektieren, die für die Atmungszyklen des Individuums repräsentativ sind, wobei das Verfahren ferner die Schritte aufweist:

   /a3/ Empfangen von Atmungszyklussignalen und Bestimmen einer Atmungsfrequenz (BR),
   /b3/ an der Steuereinrichtung, Extrahieren einer durchschnittlichen Atmungsfrequenz ABR(i) während des interessierenden täglichen Zeitraums T(i), wobei der Zeitraum in einem 24-Stunden-Zeitraum enthalten ist,
   /c3/ Bestimmen einer Kurve durchschnittlicher Atmungsfrequenz über die vergangenen Tage,
   /d3/ Berechnen eines charakteristischen Merkmals in der Kurve durchschnittlicher Atmungsfrequenz.

6. Verfahren nach Anspruch 5, wobei vor Schritt /d3/ vorgesehen ist:

   /s3/ Normieren der Kurve durchschnittlicher Atmungsfrequenz gegen einen Langzeitdurchschnitt LTBR, der für das menschliche Individuum spezifisch ist,
   und Schritt /d3/ ferner dadurch definiert ist, dass:
   die Kurve durchschnittlicher Atmungsfrequenz die normierte Kurve durchschnittlicher Atmungsfrequenz ist.

7. Verfahren nach einem der Ansprüche 1 bis 6, wobei in Schritt /b1/ die minimale Herzfrequenz MSHR(i) als der niedrigste Wert der Herzfrequenz während der Tiefschlafphase(n) der abgelaufenen Nacht genommen wird.

8. Verfahren nach einem der Ansprüche 5 bis 6, wobei in Schritt /b3/ die durchschnittliche Atmungsfrequenz BR(i) als der niedrigste Wert der Atmungsfrequenz während der Tiefschlafphase(n) der abgelaufenen Nacht genommen wird.

9. Verfahren nach einem der Ansprüche 5 bis 6, wobei in Schritt /e/ der Ovulationswahrscheinlichkeitsindex als eine gewichtete Kombination von charakteristischen Merkmalen berechnet wird, die aus jeder dazugehörigen Kurve berechnet sind.

10. Verfahren nach einem der Ansprüche 1 bis 9, wobei der interessierende tägliche Zeitraum definiert ist, um eine Schlafphase des Individuums zu sein, wobei die Schlafphase bestimmt ist, wenn eine von einem Bewegungssensor detektierte Bewegung unterhalb eines vorgegebenen Pegels (MTH) ist.

11. Verfahren nach einem der Ansprüche 1 bis 10, wobei der eine oder mehr nichtinvasive Sensor eine am Handgelenk getragene Vorrichtung aufweist, die in Kontakt oder gegenüber eines Abschnitts der Haut des Individuums angeordnet ist und eine photoplethysmographische Technik verwendet, die konfiguriert ist, Herzpulse des Individuums zu messen.

12. Vorrichtung, um einem weiblichen menschlichen Individuum (U) ein Fruchtbarkeitszeitfenster zu melden, wobei die Vorrichtung konfiguriert ist, das Verfahren nach einem der Ansprüche 1 bis 11 auszuführen.

13. Vorrichtung nach Anspruch 12, wobei der eine oder mehr nichtinvasive Sensor eine messende Matte in einem Bett (19) aufweist.

14. Vorrichtung nach einem der Ansprüche 12 bis 13, wobei der eine oder mehr nichtinvasive Sensor eine Videokamera (18) aufweist.

**Revendications**

1. Procédé pour déterminer une période de fertilité pour un individu humain féminin (U), réalisé dans un système comprenant un dispositif de commande (2), un ou plusieurs capteurs non invasifs (4) configurés pour détecter des signaux de pulsation cardiaque de l'individu, au moins durant le sommeil de l'individu, le procédé comprenant les étapes suivantes :

/a1/ la réception des signaux de pulsation cardiaque et la détermination d'une fréquence cardiaque (HR),
/b1/ l'extraction, au niveau du dispositif de commande, d'une fréquence cardiaque minimale MSHR(i), sur une période de temps quotidienne d'intérêt T(i), ladite période de temps d'intérêt étant comprise dans une période de 24 heures, i désignant un indice quotidien,
/c1/ la détermination, avec des valeurs sur les derniers jours, d'une courbe de fréquence cardiaque minimale (58),
/d1/ le calcul d'une propriété caractéristique dans la courbe de fréquence cardiaque minimale,
/e/ la déduction d'un indice de probabilité d'ovulation actuel, en fonction d'au moins la propriété caractéristique dans la courbe de fréquence cardiaque minimale,
/f/ si l'indice de probabilité d'ovulation est supérieur à un seuil prédéfini désigné par PTH, la notification à l'individu d'une fenêtre de temps de fertilité.

2. Procédé selon la revendication 1, dans lequel avant l'étape /d1/ a lieu :

/s1/ la normalisation de la courbe de fréquence cardiaque minimale versus une moyenne de fréquence cardiaque à long terme LTHR spécifique à l'individu humain,
et l'étape /d1/ est en outre définie par :
la courbe de fréquence cardiaque minimale est la courbe de fréquence cardiaque minimale normalisée.

3. Procédé selon la revendication 1 ou la revendication 2,
le procédé comprenant en outre les étapes suivantes :

/a2/ la détermination, à partir d'une pluralité de signaux de pulsation cardiaque, d'une ou plusieurs valeurs de variabilité de fréquence cardiaque (HRV),

/b2/ l'extraction, au niveau du dispositif de commande, d'un ou plusieurs indices de HRV désignés par HRVInd(i), sur la période de temps quotidienne d'intérêt T(i),

/c2/ la détermination, avec des valeurs sur les derniers jours, d'une ou plusieurs courbes d'indices de HRV,

/d2/ le calcul d'une propriété caractéristique dans les une ou plusieurs courbes d'indices de HRV.

4. Procédé selon la revendication 3, dans lequel avant l'étape /d2/ a lieu :

/s2/ la normalisation de chacune des courbes versus une moyenne à long terme (LTHRV) spécifique à l'individu humain,

et l'étape /d2/ est en outre définie par :

les une ou plusieurs courbes d'indices de HRV sont les une ou plusieurs courbes d'indices de HRV normalisées.

5. Procédé selon l'une quelconque des revendications 1 à 4, dans lequel les un ou plusieurs capteurs non invasifs (4) sont configurés pour détecter des signaux de respiration représentatifs des cycles de respiration de l'individu, le procédé comprenant les étapes suivantes :

/a3/ la réception de signaux de cycle de respiration et la détermination d'une fréquence respiratoire (BR),

/b3/ l'extraction, au niveau du dispositif de commande, d'une fréquence respiratoire moyenne ABR(i), durant la période de temps quotidienne d'intérêt T(i), ladite période de temps étant comprise dans une période de 24 heures,

/c3/ la détermination, sur les derniers jours, d'une courbe de fréquence respiratoire moyenne,

/d3/ le calcul d'une propriété caractéristique dans la courbe de fréquence respiratoire moyenne.

6. Procédé selon la revendication 5, dans lequel avant l'étape /d3/ a lieu /s3/ la normalisation de la courbe de fréquence respiratoire moyenne versus une moyenne à long terme LTBR spécifique à l'individu humain,

et l'étape /s3/ est en outre définie par :

la courbe de fréquence respiratoire moyenne est la courbe de fréquence respiratoire moyenne normalisée.

7. Procédé selon l'une quelconque des revendications 1 à 6, dans lequel à l'étape /b1/, la fréquence cardiaque minimale MSHR(i) est prise comme valeur de fréquence cardiaque la plus basse durant la ou les phases de sommeil profond de la nuit écoulée.

8. Procédé selon l'une quelconque des revendications 5 et 6, dans lequel à l'étape /b3/, la fréquence respiratoire moyenne BR(i) est prise comme valeur de fréquence respiratoire la plus basse durant la ou les phases de sommeil profond de la nuit écoulée.

9. Procédé selon l'une quelconque des revendications 5 et 6, dans lequel à l'étape /e/, l'indice de probabilité d'ovulation est calculé comme combinaison pondérée de propriétés caractéristiques calculées à partir de chaque courbe impliquée.

10. Procédé selon l'une quelconque des revendications 1 à 9 dans lequel la période de temps quotidien d'intérêt est définie pour être une phase de sommeil de l'individu, la phase de sommeil étant déterminée lorsqu'un mouvement détecté par un capteur de mouvement est sous un niveau prédéfini (MTH).

11. Procédé selon l'une quelconque des revendications 1 à 10, dans lequel les un ou plusieurs capteurs non invasifs comprennent un dispositif porté au poignet placé en contact avec une partie de la peau de l'individu ou à l'opposé de celle-ci et utilisant une technique photopléthysmographique, configuré pour détecter des pulsations cardiaques de l'individu.

12. Appareil pour notifier un individu humain féminin (U) d'une fenêtre de temps de fertilité, configuré pour réaliser le procédé selon l'une quelconque des revendications 1 à 11.

13. Appareil selon la revendication 12, dans lequel les un ou plusieurs capteurs non invasifs comprennent une natte de détection dans un lit (19).

14. Appareil selon l'une quelconque des revendications 12 et 13, dans lequel les un ou plusieurs capteurs non invasifs comprennent une caméra vidéo (18).

**FIG. 1**

90    1

**FIG. 2**

FIG. 3

FIG. 4

★ Menstruation
▽ Ovulation
▫ MSHR(k)

FIG. 5

FIG. 6

**FIG. 7**

**FIG. 8**

FIG. 9

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015150434 A
- US 20160058428 A
- EP 2976998 A
- EP 2873368 A